(19) Europäisches Patentamt
European Patent Office
Office européen des brevets

(11) Veröffentlichungsnummer: **0 581 166 A1**

(12) # EUROPÄISCHE PATENTANMELDUNG

(21) Anmeldenummer: **93111581.0**

(22) Anmeldetag: **20.07.93**

(51) Int. Cl.5: **C07D 235/08**, C07D 235/26, C07D 471/04, C07D 417/04, C07D 403/14, C07D 417/14, A61K 31/415, A61K 31/54, A61K 31/435, //(C07D471/04, 235:00,221:00)

(30) Priorität: **22.07.92 DE 4224133**

(43) Veröffentlichungstag der Anmeldung:
**02.02.94 Patentblatt 94/05**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach(DE)**

(72) Erfinder: **Hauel, Norbert, Dr., Dipl.-Chem.**
**Marderweg 12**
**D-88433 Schemmerhofen(DE)**
Erfinder: **Ries, Uwe, Dr., Dipl.-Chem.**
**Dunantstrasse 10**
**D-88400 Biberach(DE)**
Erfinder: **Van Meel, Jacques, Dr.**
**Schubertweg 4**
**D-88441 Mittelbiberach(DE)**
Erfinder: **Wienen, Wolfgang, Dr., Dipl.-Biol.**
**Am Schiessberg 28**
**D-88437 Äpfingen(DE)**
Erfinder: **Entzeroth, Michael, Dipl.-Chem., Dr.**
**Sebastian-Sailer-Strasse 44**
**D-88447 Warthausen(DE)**

(54) **Benzimidazole, Verfahren zu ihrer Herstellung und deren Verwendung als Angiotensin-II-Antagonisten.**

(57) Die Erfindung betrifft Benzimidazole der allgemeinen Formel

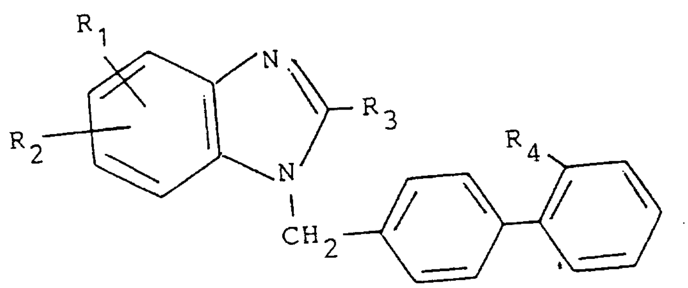

,(I)

in der
$R_1$ bis $R_4$ wie im Anspruch 1 definiert sind, deren 1-, 3-Isomerengemische und deren Salze, welche wertvolle Eigenschaften aufweisen.

Die neuen Verbindungen stellen insbesondere Angiotensin-Antagonisten dar.

EP 0 581 166 A1

Gegenstand der vorliegenden Erfindung sind neue Benzimidazole der allgemeinen Formel

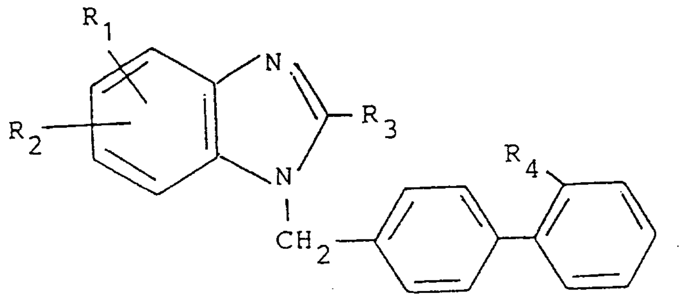

, (I)

deren Tautomere und deren Salze, insbesondere für die pharmazeutische Anwendung deren physiologisch verträglichen Salze mit anorganischen oder organischen Säuren oder Basen, welche wertvolle Angiontensin-Antagonisten, insbesondere Angiontensin-II-Antagonisten,darstellen, diese Verbindungen enthaltende Arzneimittel, deren Verwendung und Verfahren zu ihrer Herstellung.

In der obigen allgemeinen Formel I bedeutet

$R_1$ ein Wasserstoff-, Fluor-, Chlor- oder Bromatom, eine Alkylgruppe mit 1 bis 3 Kohlenstoffatomen, eine Cycloalkylgruppe mit 3 bis 7 Kohlenstoffatomen, eine Fluormethyl-, Difluormethyl- oder Trifluormethylgruppe,

$R_2$ eine Phthalimino- oder Homophthaliminogruppe, wobei eine Carbonylgruppe in einer Phthaliminogruppe durch eine Methylengruppe ersetzt sowie eine Methylengruppe in einer Homophthaliminogruppe durch eine oder zwei Alkylgruppen mit jeweils 1 bis 3 Kohlenstoffatomen substituiert sein kann,

eine gegebenenfalls durch eine oder zwei Alkylgruppen mit jeweils 1 bis 3 Kohlenstoffatomen substituierte 5-, 6- oder 7-gliedrige Alkyleniminogruppe, in welcher eine Methylengruppe durch eine Carbonyl- oder Sulfonylgruppe ersetzt ist,

eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,

eine gegebenenfalls in 1-Stellung durch eine Alkylgruppe mit 1 bis 6 Kohlenstoffatomen oder eine Cycloalkylgruppe mit 3 bis 5 Kohlenstoffatomen substituierte Benzimidazol-2-yl- oder 4,5,6,7-Tetrahydrobenzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann, eine Imidazo-[2,1-b]thiazol-6-yl-, Imidazo[1,2-a]pyridin-2-yl-, 5,6,7,8-Tetrahydroimidazo[1,2-a]pyridin-2-yl-, 3-Chlor-5,6,7,8-tetrahydroimidazo[1,2-a]pyridin-2-yl-, Imidazo[1,2-a]pyrimidin-2-yl-, Imidazo[4,5-b]pyridin-2-yl-, Imidazo[4,5-c]pyridin-2-yl-, Imidazo[1,2-c]pyrimidin-2-yl-, Imidazo[1,2-a]pyrazin-2-yl-, Imidazo[1,2-b]pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl- oder Imidazo[4,5-d]pyridazin-2-yl-gruppe, einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonylgruppe ersetzt sein kann, oder

eine $R_7$-$NR_6$-CO-$NR_5$-Gruppe, in der

$R_5$ ein Wasserstoffatom, eine Alkylgruppe mit 1 bis 5 Kohlenstoffatomen, eine Cyclohexyl- oder Benzylgruppe,

$R_6$ ein Wasserstoffatom, eine Alkylgruppe mit 1 bis 6 Kohlenstoffatomen, eine Allyl-, Cyclohexyl-, Benzyl- oder Phenylgruppe,

$R_7$ ein Wasserstoffatom oder eine Alkylgruppe mit 1 bis 3 Kohlenstoffatomen oder

$R_6$ und $R_7$ zusammen mit dem dazwischen liegenden Stickstoffatom eine unverzweigte cyclische Alkyleniminogruppe mit 4 bis 6 Kohlenstoffatomen oder eine Morpholinogruppe oder

$R_5$ und $R_6$ zusammen eine Alkylengruppe mit 2 oder 3 Kohlenstoffatomen darstellen,

$R_3$ eine Alkylgruppe mit 1 bis 5 Kohlenstoffatomen, eine Cycloalkylgruppe mit 3 bis 5 Kohlenstoffatomen oder eine Alkoxygruppe mit 1 bis 3 Kohlenstoffatomen und

$R_4$ eine in 1- oder 2-Stellung durch eine $R_a$-CO-O-$CH_2$-Gruppe substituierte Tetrazolylgruppe, eine $R_b$-CO-O-($R_c$CH)-O-CO-, $R_a$O-CO- oder $R_b$O-CO-O-($R_c$CH)-O-CO-Gruppe, wobei

$R_a$ eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,

$R_b$ eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe und

$R_c$ ein Wasserstoffatom oder eine Methylgruppe darstellen,

oder auch, wenn

(i) $R_1$ und $R_2$ wie vorstehend erwähnt definiert sind und $R_3$ eine Alkoxygruppe darstellt, eine Carboxy-, 1H-Tetrazolyl- oder 2H-Tetrazolylgruppe oder, wenn

(ii) $R_1$ wie vorstehend erwähnt definiert ist, $R_2$ mit Ausnahme der 1-Methyl-benzimidazol-2-yl-gruppe wie vorstehend erwähnt definiert ist und $R_3$ eine Cyclopropylgruppe darstellt, eine Carboxygruppe oder, wenn

(iii) $R_1$ und $R_3$ wie vorstehend erwähnt definiert sind und $R_2$ eine Butansultam-1-yl-gruppe darstellt, eine Carboxygruppe oder, wenn

(iv) $R_1$ wie vorstehend erwähnt definiert ist, $R_2$ eine 1-Methyl-5-fluor-benzimidazol-2-yl-gruppe und $R_3$ eine Ethylgruppe darstellen, eine Carboxy-, 1H-Tetrazolyl- oder 2H-Tetrazolylgruppe.

Für die bei der Definition der Reste $R_1$ bis $R_4$ eingangs erwähnten Bedeutungen kommt beispielsweise für

$R_1$ die Bedeutung des Wasserstoffatoms oder in 4-Position die des Fluor-, Chlor- oder Bromatoms, der Methyl-, Ethyl-, n-Propyl-, Isopropyl-, Cyclopropyl-, Cyclobutyl-, Cyclopentyl-, Cyclohexyl-, Cycloheptyl-, Difluormethyl- oder eine Trifluormethylgruppe,

$R_2$ die der Phthalimino-, Homophthalimino-, 4,4-Dimethylhomophthalimino-, 1-Oxo-isoindolin-2-yl-, 2-Oxo-pyrrolidino-, 2-Oxo-piperidino-, 2-Oxo-hexamethylenimino, Propansultam-1-yl-, Butansultam-1-yl-, Pentansultam-1-yl-, Maleinsäureimido-, 2-Methyl-maleinsäureimido-, 2-Phenyl-maleinsäureimido-, 2,3-Dimethyl-maleinsäureimido, 3-Methyl-2-phenyl-maleinsäureimido-, 2,3-Diphenyl-maleinsäureimido-, Piperidin-2-yl-, Piperidin-2-on-1-yl-, Chinolin-2-yl-, Isochinolin-1-yl-, Isochinolin-3-yl-, Pyridin-2-yl-, Benzimidazol-2-yl-, 1-Methylbenzimidazol-2-yl-, 1-Ethyl-benzimidazol-2-yl-, 1-n-Propyl-benzimidazol-2-yl-, 1-Isopropyl-benzimidazol-2-yl-, 1-n-Butyl-benzimidazol-2-yl-, 1-Isobutyl-benzimidazol-2-yl-, 1-n-Pentyl-benzimidazol-2-yl-, 1-n-Hexyl-benzimidazol-2-yl-, 1-Cyclopropyl-benzimidazol-2-yl-, 1-Cyclobutyl-benzimidazol-2-yl-,1-Cyclopentyl-benzimidazol-2-yl-, 1-Cyclohexyl-benzimidazol-2-yl-,5-Fluor-1-methyl-benzimidazol-2-yl-, 6-Fluor-1-methylbenzimidazol-2-yl-, 5-Trifluormethyl-benzimidazol-2-yl-, 5-Trifluormethyl-1-methyl-benzimidazol-2-yl-, Imidazo[1,2-a]pyridin-2-yl-, 5,6,7,8-Tetrahydro-imidazo[1,2-a]pyridin-2-yl-, 3-Chlor-5,6,7,8-tetrahydroimidazo[1,2-a]pyridin-2-yl-, Imidazo[1,2-a]pyrimidin-2-yl-, Imidazo[4,5-b]pyridin-2-yl-, Imidazo[4,5-c]pyridin-2-yl-, Imidazo[2,1-b]thiazol-6-yl-, Imidazo[1,2-c]pyrimidin-2-yl-, Imidazo[1,2-a]pyrazin-2-yl-, Imidazo[1,2-b]-pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl-, Imidazo[4,5-d]pyridazin-2-yl-, Aminocarbonylamino-, Methylaminocarbonylamino-, Dimethylaminocarbonylamino-, N-Methylaminocarbonyl-methylamino-, N-(Dimethylaminocarbonyl)-methylamino-,N-Dimethylaminocarbonylethylamino-, N-Dimethylaminocarbonyl-isopropylamino-, N-(Dimethylaminocarbonyl)-n-pentylamino-, N-Methylaminocarbonylethylamino-, N-Methylaminocarbonyl-n-pentylamino-, N-Methylaminocarbonyl-n-hexylamino-, N-Methylaminocarbonyl-n-octylamino-, N-Methylaminocarbonyl-cyclohexylamino-, Ethylaminocarbonylamino-, N-Ethylaminocarbonyl-methylamino-, N-Ethylaminocarbonyl-ethylamino-, N-Ethylaminocarbonyl-n-hexylamino-, N-Ethylaminocarbonyl-n-heptylamino-, N-Ethylaminocarbonylcyclohexylamino-, Diethylaminocarbonylamino-, N-(Diethylaminocarbonyl)-methylamino-, N-(Diethylaminocarbonyl)-ethylamino-, N-(Diethylaminocarbonyl)-n-butylamino-, N-(Diethylaminocarbonyl)-n-hexylamino-, N-(Diethylaminocarbonyl)-n-octylamino-, Isopropylaminocarbonylamino-, N-Isopropylaminocarbonyl-methylamino-, n-Butylaminocarbonylamino-, N-(n-Butylaminocarbonyl)-methylamino-, N-(n-Butylaminocarbonyl)-ethylamino-, N-(n-Butylaminocarbonyl)-isopropylamino-, N-(n-Butylaminocarbonyl)-n-butylamino-, N-(n-Butylaminocarbonyl)-n-hexylamino-, N-(n-Butylaminocarbonyl)-cyclohexylamino-, N-(Di-(n-Butyl)-aminocarbonyl)-amino-, N-(Di-(n-Butyl)-aminocarbonyl)-methylamino-, N-(Di-(n-Butyl)-aminocarbonyl)-ethylamino-, N-(Di-(n-Butyl)-aminocarbonyl)-n-butylamino-, N-(Di-(n-Butyl)-aminocarbonyl)-n-hexylamino-, N-(n-Pentylaminocarbonyl)-methylamino-, N-(n-Pentylaminocarbonyl)-ethylamino-, N-(n-Hexylaminocarbonyl)-ethylamino-, n-Hexylaminocarbonylamino-, n-Heptylaminocarbonylamino-, n-Octylaminocarbonylamino-, N-(n-Hexylaminocarbonyl)-n-butylamino-, N-(n-Hexylaminocarbonyl)-n-pentylamino-, N-(n-Hexylaminocarbonyl)-n-hexylamino-, N-(n-Hexylaminocarbonyl)-cyclohexylamino-, Di-(n-Hexyl)-aminocarbonylamino-, N-(Di-(n-Hexyl)-aminocarbonyl)-methylamino-, N-((n-Hexyl)-methylaminocarbonyl)-amino-, Cyclohexylaminocarbonylamino-, N-Cyclohexylaminocarbonyl-methylamino-, N-Cyclohexylaminocarbonyl-ethylamino-, N-Cyclohexylaminocarbonyl-n-butylamino-, N-Cyclohexylaminocarbonyl-isobutylamino-, N-Cyclohexylaminocarbonyl-n-pentylamino-, N-Cyclohexylaminocarbonyl-n-hexylamino-, N-Cyclohexylaminocarbonyl-cyclohexylamino-, N-(Ethyl-cyclohexylaminocarbonyl)-methylamino-, N-(Propyl-cyclohexylaminocarbonyl)-methylamino-, N-(n-Butyl-cyclohexylaminocarbonyl)-methylamino-, Allylaminocarbonylamino, Benzylaminocarbonylamino-, N-Benzylaminocarbonyl-isobutylamino-, Phenylaminocarbonylamino-, Pyrrolidinocarbonylamino-, Pyrrolidinocarbonylmethylamino-, Piperidinocarbonylamino-, Hexamethyleniminocarbonylamino-, Morpholinocarbonylamino-, 3,4,5,6-Tetrahydro-2-pyrimidon-1-yl-, 3-Methyl-3,4,5,6-tetrahydro-2-pyrimidon-1-yl-, 3-Ethyl-3,4,5,6-tetrahydro-2-pyrimidon-1-yl-, 3-n-Propyl-3,4,5,6-tetrahydro-2-pyrimidon-1-yl-, 3-Isopropyl-3,4,5,6-tetrahydro-2-pyrimidon-1-yl-, 3-n-Butyl-3,4,5,6-tetrahydro-2-pyrimidon-1-yl, 3-Isobutyl-3,4,5,6-tetrahydro-2-pyrimidon-1-yl-, 3-n-Pentyl-3,4,5,6-tetrahydro-2-pyrimidon-1-yl-, 3-n-Hexyl-3,4,5,6-tetrahydro-2-pyrimidon-1-yl-, 3-Cyclopentyl-3,4,5,6-tetrahydro-2-pyrimidon-1-yl-, 3-Cyclohexyl-3,4,5,6-tetrahydro-2-pyrimidon-1-yl-, 3-Cycloheptyl-3,4,5,6-tetrahydro-2-pyrimidon-1-yl-, 3-Benzyl-3,4,5,6-tetrahydro-2-pyrimidon-1-yl-, 3,4,5,6-Tetrahydro-2(1H)-pyrimidon-1-yl-, 3-Methyl-3,4,5,6-tetrahydro-2(1H)-pyrimidon-1-yl-, 3-Ethyl-3,4,5,6-tetrahydro-2(1H)-pyrimidon-1-yl-, 3-n-Propyl-3,4,5,6-tetrahydro-2(1H)-pyrimidon-1-yl-, 3-Isopropyl-3,4,5,6-tetrahydro-2(1H)-pyrimidon-1-yl-, 3-Benzyl-3,4,5,6-tetrahydro-2(1H)-pyrimidon-1-yl-, 3-(2-Phenylethyl)-3,4,5,6-tetrahydro-2(1H)-pyrimidon-1-yl- oder 3-(3-Phenylpropyl)-3,4,5,6-tetra-hydro-2(1H)-pyrimidon-1-yl-gruppe,

für $R_3$ die der Methyl-, Ethyl-, n-Propyl-, Isopropyl-, n-Butyl-, Isobutyl-, tert.Butyl-, n-Pentyl-, 1-Methyl-butyl-, 2-Methyl-butyl-, 3-Methyl-butyl-, Cyclopropyl-, Cyclobutyl-, Cyclopentyl-, Methoxy-, Ethoxy-, n-Propoxy- oder Isopropoxygruppe und

für $R_4$ die der Hydroxycarbonyl-, Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, Isoamyloxycarbonyl-, n-Hexyloxycarbonyl-, Cyclopentyloxycarbonyl-, Cyclohexyloxycarbonyl-, Benzyloxycarbonyl-, 1-Phenylethyloxycarbonyl-, 2-Phenylethyloxycarbonyl-, 3-Phenylpropyloxycarbonyl-, Methoxymethoxycarbonyl-, Cinnamyloxycarbonyl-, Acetoxymethoxycarbonyl-, Propionyloxymethoxycarbonyl-, n-Butyryloxymethoxycarbonyl-, Isobutyryloxymethoxycarbonyl-, n-Pentanoyloxymethoxycarbonyl-, Isopentanoyloxymethoxycarbonyl-, Pivaloyloxymethoxycarbonyl-, n-Hexanoyloxymethoxycarbonyl-, Cyclopentanoyloxymethoxycarbonyl-, Cyclohexanoyloxymethoxycarbonyl-, Phenylacetoxymethoxycarbonyl-, 2-Phenylpropionyloxymethoxycarbonyl-, 3-Phenylpropionyloxymethoxycarbonyl-, 4-Phenylbutyryloxymethoxycarbonyl-, Benzoyloxymethoxycarbonyl-, 1-Acetoxyethoxycarbonyl-, 1-Propionyloxyethoxycarbonyl-, 1-n-Butyryloxyethoxycarbonyl-, 1-Isobutyryloxyethoxycarbonyl-, 1-n-Pentanoyloxyethoxycarbonyl-, 1-Isopentanoyloxyethoxycarbonyl-, 1-Pivaloyloxyethoxycarbonyl-, 1-n-Hexanoyloxyethoxycarbonyl-, 1-Cyclopentanoyloxyethoxycarbonyl-, 1-Cyclohexanoyloxyethoxycarbonyl-, 1-Phenylacetoxyethoxycarbonyl-, 1-(1-Phenylpropionyloxy)-ethoxycarbonyl-, 1-(2-Phenylpropionyloxy)-ethoxycarbonyl-, 1-(3-Phenylbutyryloxy)-ethoxycarbonyl-, 1-Benzoyloxyethoxycarbonyl-, Methoxycarbonyloxymethoxycarbonyl-, Ethoxycarbonyloxymethoxycarbonyl-, n-Propyloxycarbonyloxymethoxycarbonyl-, Isopropyloxycarbonyloxymethoxycarbonyl-, n-Butyloxycarbonyloxymethoxycarbonyl-,Isobutyloxycarbonyloxymethoxycarbonyl-, tert.Butyloxycarbonyloxymethoxycarbonyl-, n-Pentyloxycarbonyloxymethoxycarbonyl-,Isoamyloxycarbonyloxymethoxycarbonyl-, n-Hexyloxycarbonyloxymethoxycarbonyl-, Cyclopentyloxycarbonyloxymethoxycarbonyl-,Cyclohexyloxycarbonyloxymethoxycarbonyl-, Benzyloxycarbonyloxymethoxycarbonyl-, 1-Phenylethoxycarbonyloxymethoxycarbonyl-, 2-Phenylethoxycarbonyloxymethoxycarbonyl-, 3-Phenylpropyloxycarbonyloxymethoxycarbonyl-, Cinnamyloxycarbonyloxymethoxycarbonyl-, 1-(Methoxycarbonyloxy)-ethoxycarbonyl-, 1-(Ethoxycarbonyloxy)-ethoxycarbonyl-, 1-(n-Propyloxycarbonyloxy)-ethoxycarbonyl-, 1-(Isopropyloxycarbonyloxy)-ethoxycarbonyl-, 1-(n-Butyloxycarbonyloxy)-ethoxycarbonyl-, 1-(Isobutyloxycarbonyloxy)-ethoxycarbonyl-, 1-(tert.Butyloxycarbonyloxy)-ethoxycarbonyl-, 1-(n-Pentyloxycarbonyloxy)-ethoxycarbonyl, 1-(Isoamyloxycarbonyloxy)-ethoxycarbonyl-, 1-(n-Hexyloxycarbonyloxy)-ethoxycarbonyl-, 1-(Cyclopentyloxycarbonyloxy)-ethoxycarbonyl-, 1-(Cyclohexyloxycarbonyloxy)-ethoxycarbonyl-, Cyclopentylcarbonyloxymethoxycarbonyl-, 1-(Benzyloxycarbonyloxy)-ethoxycarbonyl-, 1-(1-Phenylethoxycarbonyloxy)-ethoxycarbonyl-, 1-(2-Phenylethoxycarbonyloxy)-ethoxycarbonyl-, 1-(3-Phenylpropyloxycarbonyloxy)-ethoxycarbonyl-, 1-(Cinnamyloxycarbonyloxy)-ethoxycarbonyl-, Cyano-, 1H-Tetrazolyl-, 1-Triphenylmethyl-tetrazolyl-, 2-Triphenylmethyl-tetrazolyl-, 1-Acetoxymethyl-tetrazolyl-, 2-Acetoxymethyl-tetrazolyl-, 1-Propionyloxymethyl-tetrazolyl-, 2-Propionyloxymethyl-tetrazolyl-, 1-Butyryloxymethyl-tetrazolyl-, 2-Butyryloxymethyl-tetrazolyl-, 1-Isobutyryloxymethyl-tetrazolyl-, 2-Isobutyryloxymethyl-tetrazolyl-, 1-Pivaloyloxymethyl-tetrazolyl-, 2-Pivaloyloxymethyl-tetrazolyl-, 1-Ethoxycarbonyloxymethyl-tetrazolyl-, 2-Ethoxycarbonyloxymethyl-tetrazolyl-, 1-[1-(Ethoxycarbonyloxy)-ethyl]-tetrazolyl-, 2-[1-(Ethoxycarbonyloxy)-ethyl]-tetrazolyl-, 1-[1-(Cyclohexyloxycarbonyloxy)-ethyl]-tetrazolyl- oder 2-[1-(Cyclohexyloxycarbonyloxy)-ethyl]-tetrazolyl-gruppe in Betracht.

Besonders bevorzugte Verbindungen der obigen allgemeinen Formel I sind diejenigen, in denen

$R_1$ ein Wasserstoffatom oder in 4-Position ein Chloratom, eine Alkylgruppe mit 1 bis 3 Kohlenstoffatomen oder eine Trifluormethylgruppe,

$R_2$ eine Phthalimino- oder Homophthaliminogruppe, wobei eine Carbonylgruppe in einer Phthaliminogruppe durch eine Methylengruppe ersetzt sein kann,

eine 5-, 6- oder 7-gliedrige Alkyleniminogruppe, in welcher eine Methylengruppe durch eine Carbonyl- oder

Sulfonylgruppe ersetzt ist,
eine gegebenenfalls durch eine Methyl- oder Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom eine Methyl- oder Trifluormethylgruppe substituiert sein kann, eine Imidazo[2,1-b]thiazol-6-yl-, Imidazo[1,2-a]-pyridin-2-yl-, 5,6,7,8-Tetrahydro-imidazo[1,2-a]pyridin-2-yl-, 3-Chlor-5,6,7,8-tetrahydro-imidazo[1,2-a]pyridin-2-yl-, Imidazo[1,2-a]pyrimidin-2-yl-, Imidazo[4,5-b]pyridin-2-yl-, Imidazo[4,5-c]pyridin-2-yl-, Imidazo[1,2-c]-pyrimidin-2-yl-, Imidazo[1,2-a]pyrazin-2-yl-, Imidazo[1,2-b]pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl- oder Imidazo[4,5-d]pyridazin-2-yl-gruppe oder
eine $R_7$-$NR_6$-CO-$NR_5$-Gruppe, in der
$R_5$ ein Wasserstoffatom, eine Alkylgruppe mit 1 bis 5 Kohlenstoffatomen, eine Cyclohexyl- oder Benzylgruppe,
$R_6$ ein Wasserstoffatom, eine Alkylgruppe mit 1 bis 6 Kohlenstoffatomen, eine Allyl-, Cyclohexyl-, Benzyl- oder Phenylgruppe,
$R_7$ ein Wasserstoffatom oder eine Alkylgruppe mit 1 bis 3 Kohlenstoffatomen oder
$R_6$ und $R_7$ zusammen mit dem dazwischen liegenden Stickstoffatom eine geradkettige Alkyleniminogruppe mit 4 bis 6 Kohlenstoffatomen oder eine Morpholinogruppe oder
$R_5$ und $R_6$ zusammen eine Alkylengruppe mit 2 oder 3 Kohlenstoffatomen darstellen,
$R_3$ eine Alkylgruppe mit 1 bis 5 Kohlenstoffatomen, eine Cycloalkylgruppe mit 3 bis 5 Kohlenstoffatomen oder eine Alkoxygruppe mit 2 bis 3 Kohlenstoffatomen und
$R_4$ eine in 1- oder 2-Stellung durch eine $R_a$-CO-O-$CH_2$-Gruppe substituierte Tetrazolylgruppe, eine $R_b$-CO-O-($R_c$CH)-O-CO-, $R_a$O-CO- oder $R_b$O-CO-O-($R_c$CH)-O-CO-Gruppe, wobei
$R_a$ eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
$R_b$ eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe und
$R_c$ ein Wasserstoffatom oder eine Methylgruppe darstellen,
oder auch, wenn
(i) $R_1$ und $R_2$ wie vorstehend erwähnt definiert sind und $R_3$ eine Alkoxygruppe darstellt, eine Carboxy-, 1H-Tetrazo-1yl- oder 2H-Tetrazolylgruppe oder, wenn
(ii) $R_1$ wie vorstehend erwähnt definiert ist, $R_2$ mit Ausnahme der 1-Methyl-benzimidazol-2-yl-gruppe wie vorstehend erwähnt definiert ist und $R_3$ eine Cyclopropylgruppe darstellt, eine Carboxygruppe oder, wenn
(iii) $R_1$ und $R_3$ wie vorstehend erwähnt definiert sind und $R_2$ eine Butansultam-1-yl-gruppe darstellt, eine Carboxygruppe oder, wenn
(iv) $R_1$ wie vorstehend erwähnt definiert ist, $R_2$ eine 1-Methyl-5-fluor-benzimidazol-2-yl-gruppe und $R_3$ eine Ethylgruppe darstellen, eine Carboxy-, 1H-Tetrazolyl- oder 2H-Tetrazolylgruppe bedeuten,
deren Tautomere und deren Salze.

Ganz besonders bevorzugte Verbindungen der obigen allgemeinen Formel I sind diejenigen, in denen
$R_1$ ein Wasserstoffatom oder in 4-Position eine Methylgruppe,
$R_2$ eine Imidazo[1,2-a]pyridin-2-yl-, 5,6,7,8-Tetrahydro-imidazo[1,2-a]pyridin-2-yl-, 3-Chlor-5,6,7,8-tetrahydro-imidazo[1,2-a]pyridin-2-yl-, 1-Methyl-benzimidazol-2-yl-, 1-Methyl-5-fluor-benzimidazol-2-yl- oder Butansultam-1-yl-gruppe,
$R_3$ eine Alkylgruppe mit 2 bis 4 Kohlenstoffatomen, eine Cyclopropylgruppe oder eine Alkoxygruppe mit 2 oder 3 Kohlenstoffatomen und
$R_4$ eine in 1- oder 2-Stellung durch eine $R_a$-CO-O-$CH_2$-Gruppe substituierte Tetrazolylgruppe, eine $R_b$-CO-O-($R_c$CH)-O-CO-,
$R_a$O-CO- oder $R_b$O-CO-O-($R_c$CH)-O-CO-Gruppe bedeuten, wobei
$R_a$ eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
$R_b$ eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe und

$R_c$ ein Wasserstoffatom oder eine Methylgruppe bedeuten,

sowie die Verbindungen

4'-[(2-Cyclopropyl-4-methyl-6-(4,5,6,7-tetrahydro-imidazo[1,2-a]pyridin-2-yl)-benzimidazol-1-yl)-methyl]-biphenyl-2-carbonsäure,

4'-[(2-Ethoxy-4-methyl-6-(4,5,6,7-tetrahydro-imidazo[1,2-a]pyridin-2-yl)-benzimidazol-1-yl)-methyl]-biphenyl-2-carbonsäure,

4'-[(2-Ethoxy-4-methyl-6-(4,5,6,7-tetrahydro-imidazo[1,2-a]pyridin-2-yl)-benzimidazol-1-yl)-methyl]-2-(1H-tetrazol-5-yl)-biphenyl,

4'-[(2-Cyclopropyl-4-methyl-6-(imidazo[1,2-a]pyridin-2-yl)-benzimidazol-1-yl)-methyl]-biphenyl-2-carbonsäure,

4'-[(2-Ethoxy-4-methyl-6-(imidazo[1,2-a]pyridin-2-yl)-benzimidazol-1-yl)-methyl]-biphenyl-2-carbonsäure,

4'-[(2-Ethoxy-4-methyl-6-(imidazo[1,2-a]pyridin-2-yl)-benzimidazol-1-yl)-methyl]-2-(1H-tetrazol-5-yl)-biphenyl,

4'-[(2-Ethoxy-4-methyl-6-(1-methyl-benzimidazol-2-yl)-benzimidazol-1-yl)-methyl]-2-(1H-tetrazol-5-yl)-biphenyl,

4'-[(2-Ethyl-4-methyl-6-(1-methyl-5-fluor-benzimidazol-2-yl)-benzimidazol-1-yl)-methyl]-2-(1H-tetrazol-5-yl)-biphenyl,

4'-[(2-Ethyl-4-methyl-6-(1-methyl-5-fluor-benzimidazol-2-yl)-benzimidazol-1-yl)-methyl]-biphenyl-2-carbonsäure und

4'-[(2-n-Propyl-4-methyl-6-(butansultam-1-yl)-benzimidazol-1-yl)-methyl]-biphenyl-2-carbonsäure,

deren Tautomere und deren Salze.

Besonders bevorzugt sind folgende Verbindungen:

4'-[(2-Ethyl-4-methyl-6-(1-methyl-5-fluor-benzimidazol-2-yl)-benzimidazol-1-yl)-methyl]-biphenyl-2-carbonsäure,

4'-[(2-n-Propyl-4-methyl-6-(butansultam-1-yl)-benzimidazol-1-yl)-methyl]-biphenyl-2-carbonsäure,

4'-[(2-Cyclopropyl-4-methyl-6-(imidazo[1,2-a]pyridin-2-yl)-benzimidazol-1-yl)-methyl]-biphenyl-2-carbonsäure,

4'-[(2-Cyclopropyl-4-methyl-6-(5,6,7,8-tetrahydro-imidazo[1,2-a]pyridin-2-yl)-benzimidazol-1-yl)-methyl]-biphenyl-2-carbonsäureund

4'-[(2-Ethoxy-4-methyl-6-(1-methyl-benzimidazol-2-yl)-benzimidazol-1-yl)-methyl]-2-(1H-tetrazol-5-yl)-biphenyl,

deren Tautomere und deren Salze.

Erfindungsgemäß erhält man die Verbindungen nach folgenden Verfahren:

a) Cyclisierung einer gegebenenfalls im Reaktionsgemisch gebildeten Verbindung der allgemeinen Formel

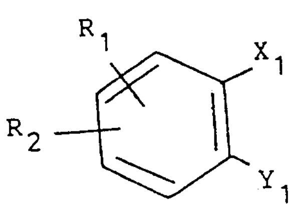 ,(II)

in der

$R_1$ und $R_2$ wie eingangs definiert sind,

einer der Reste $X_1$ oder $Y_1$ eine Gruppe der allgemeinen Formel

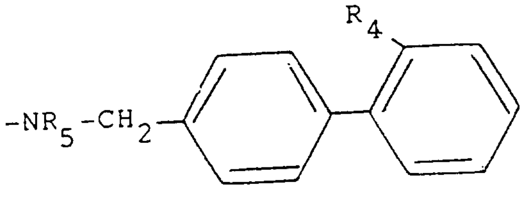

und der andere der Reste $X_1$ oder $Y_1$ eine Gruppe der allgemeinen Formel

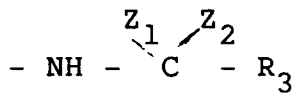

darstellen, wobei

$R_3$ und $R_4$ wie eingangs definiert sind,

$R_5$ ein Wasserstoffatom oder eine $R_3CO$-Gruppe, wobei $R_3$ wie vorstehend erwähnt definiert ist,

$Z_1$ und $Z_2$, die gleich oder verschieden sein können, gegebenenfalls durch Alkylgruppen mit 1 bis 6 Kohlenstoffatomen substituierte Amino-, Hydroxy- oder Mercaptogruppen oder $Z_1$ und $Z_2$, zusammen ein Sauerstoff- oder Schwefelatom,

eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250 °C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel II im Reaktionsgemisch durch Reduktion einer entsprechenden o-Nitro-aminoverbindung gegebenenfalls in Gegenwart einer Carbonsäure der allgemeinen Formel $R_3COOH$ oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion des erhaltenen N-Oxids der Formel I wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50 °C, vorzugsweise jedoch bei Raumtemperatur durchgeführt.

b) Umsetzung eines Benzimidazols der allgemeinen Formel

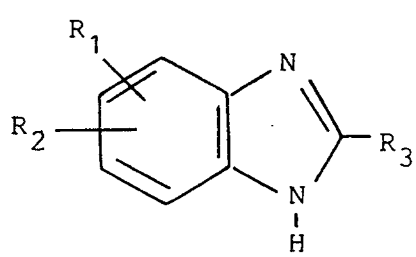

, (III)

in der

$R_1$ bis $R_3$ wie eingangs definiert sind, mit einer Biphenylverbindung der allgemeinen Formel

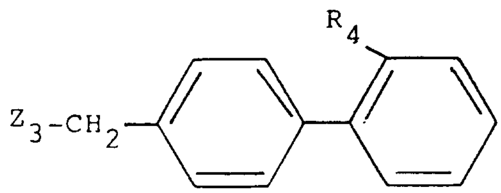

, (IV)

in der
$R_4$ wie eingangs definiert ist und
$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert.butylat, Natriumhydrid, Triethylamin oder Pyridin, wobei die beiden letzteren gleichzeitig auch als Lösungsmittel verwendet werden können, vorzugsweise bei Temperaturen zwischen 0 und 100 °C, z.B. bei Temperaturen zwischen Raumtemperatur und 50 °C, durchgeführt.

Bei der Umsetzung erhält man vorzugsweise ein Gemisch der 1- und 3-Isomeren, welches gewünschtenfalls anschließend durch Kristallisation oder chromatographisch unter Verwendung eines Trägers wie Kieselgel oder Aluminiumoxid, in das entsprechende 1- und 3-Isomere aufgetrennt wird.

c) Zur Herstellung einer Verbindung der allgemeinen Formel I, in der $R_4$ eine Carboxygruppe darstellt:
Überführung einer Verbindung der allgemeinen Formel

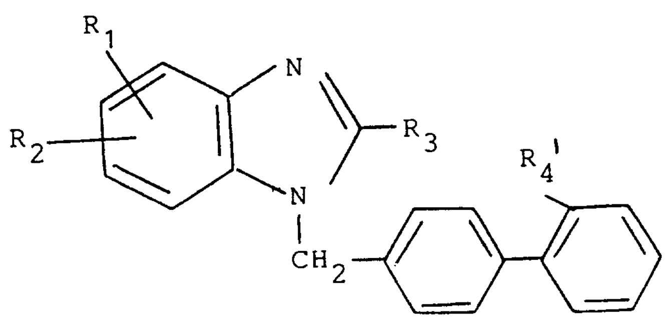

, (V)

in der
$R_1$ bis $R_3$ wie eingangs definiert sind und
$R_4$' eine mittels Hydrolyse, Thermolyse oder Hydrogenolyse in eine Carboxygruppe überführbare Gruppe darstellt.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol, Wasser/Dioxan, Methylenchlorid oder Chloroform bei Temperaturen zwischen -10 °C und 120 °C, z.B. bei Temperaturen zwischen Raumtemperatur und der Siedetemperatur des Reaktionsgemisches, durchgeführt. Bei der Hydrolyse in Gegenwart einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_4$' in einer Verbindung der allgemeinen Formel V eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei

Temperaturen zwischen O und 50°C in die Carboxygruppe übergeführt werden.

Bedeutet $R_4'$ in einer Verbindung der allgemeinen Formel V beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet $R_4'$ in einer Verbindung der allgemeinen Formel V beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

d) Zur Herstellung einer Verbindung der allgemeinen Formel I, in der $R_4$ eine 1H-Tetrazolylgruppe darstellt:

Abspaltung eines Schutzrestes von einer Verbindung der allgemeinen Formel

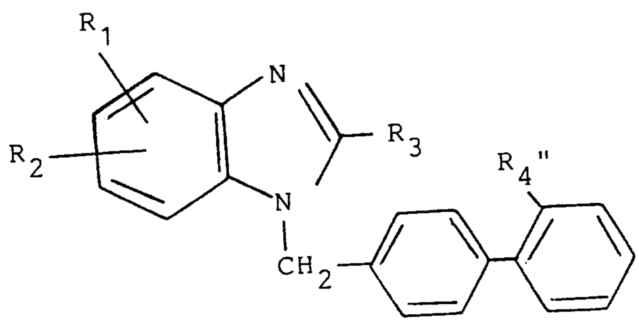

, (VI)

in der

$R_1$, $R_2$ und $R_3$ wie eingangs definiert sind und

$R_4''$ eine in 1- oder 3-Stellung durch einen Schutzrest geschützte 1H-Tetrazolylgruppe darstellt.

Als Schutzrest kommt beispielsweise die Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

e) Zur Herstellung einer Verbindung der allgemeinen Formel I, in der $R_4$ eine 1H-Tetrazolylgruppe darstellt:

Umsetzung einer Verbindung der allgemeinen Formel

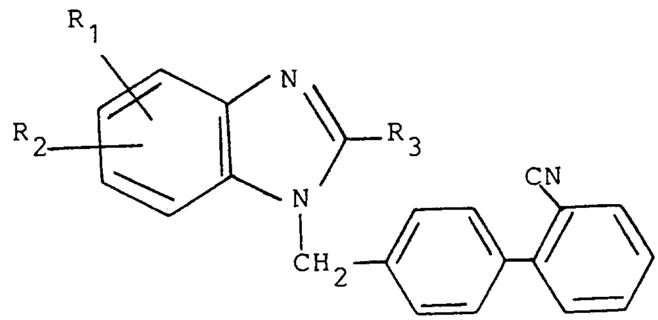

, (VII)

in der
$R_1$ bis $R_3$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150 °C, vorzugsweise bei 125 °C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

f) Zur Herstellung einer Verbindung der allgemeinen Formel I, in der $R_4$ eine in 1- oder 2-Stellung durch eine $R_a$-CO-O-$CH_2$-Gruppe substituierte Tetrazolylgruppe, eine $R_a$O-CO-, $R_b$-CO-O-($R_c$CH)-O-CO- oder $R_b$O-CO-O-($R_c$CH)-O-CO-Gruppe darstellt:
Umsetzung einer Verbindung der allgemeinen Formel

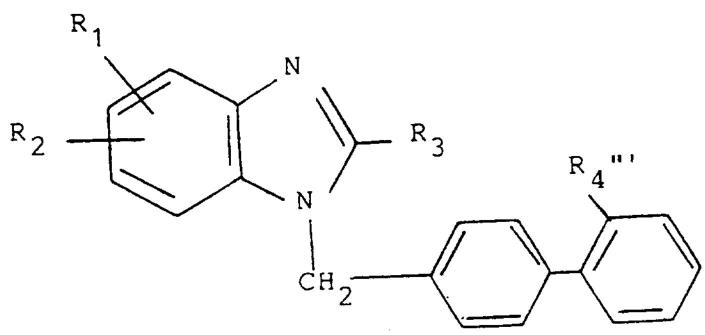

, (VIII)

in der
$R_1$ bis $R_3$ wie eingangs definiert sind und
$R_4$''' eine Carboxy-, 1H-Tetrazolyl- oder 2H-Tetrazolylgruppe bedeutet, mit einer Verbindung der allgemeinen Formel

$Z_4$ - $Y_2$ ,(IX)

in der
$Y_2$ eine $R_a$-CO-O-$CH_2$-, $R_b$-CO-O-($R_c$CH)-, $R_b$O-CO-O-($R_c$CH)- oder $R_a$-Gruppe, wobei $R_a$ bis $R_c$ wie eingangs definiert sind und $Z_4$ eine nukleophile Austrittsgruppe wie ein Halogenatom,
z. B. ein Chlor- oder Bromatom, oder auch, wenn Y eine $R_a$-Gruppe darstellt, eine Hydroxygruppe bedeuten.

Die Umsetzung erfolgt zweckmäßigerweise durch Veresterung mit einem entsprechenden Alkohol oder mit einem entsprechenden reaktionsfähigen Derivat wie dem Halogenid zweckmäßigerweise in einem Lösungsmittel oder Lösungsmittelgemisch wie Wasser, Methylenchlorid, Chloroform, Ether, Tetrahydrofuran, Dioxan oder Dimethylformamid oder in einem Überschuß des Acylierungsmittels als Lösungsmittel gegebenenfalls in Gegenwart eines die Säure aktivierenden oder wasserentziehenden Mittels wie Thionylchlorid, mit deren Anhydriden, Estern oder Halogeniden gegebenenfalls in Gegenwart einer anorganischen

oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100 °C, vorzugsweise jedoch bei Temperaturen zwischen -10 und 80 °C, durchgeführt.

Bei den vorstehend beschriebenen Umsetzungen können gegebenenfalls vorhandene reaktive Gruppen wie Hydroxy- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis IX sind teilweise literaturbekannt oder man erhält diese nach literaturbekannten Verfahren.

So erhält man beispielsweise eine Verbindung der allgemeinen Formel II durch Alkylierung einer entsprechenden o-Amino-acylamino-Verbindung mit einer Verbindung der allgemeinen Formel IV. Die hierfür erforderliche o-Amino-acylamino-Verbindung erhält man durch Reduktion einer entsprechenden o-Nitro-acylamino-Verbindung, welche man ihrerseits durch Nitrierung eines entsprechenden Acylamino-acetophenons, anschließende Überführung des erhaltenen entsprechenden o-Nitro-acylamino-acetophenons in das entsprechende ω-Brom-acetophenon, anschließende Cyclisierung des ω-Brom-acetophenons mit einem entsprechenden Säureamid und anschließende Reduktion der Nitrogruppe erhält. Vor der Reduktion der Nitrogruppe kann eine so erhaltene Oxazol-4-yl-Verbindung mittels einem entsprechenden Amin, vorzugsweise mit Ammoniak, unter Druck in die entsprechende Imidazol-4-yl-Verbindung übergeführt werden oder eine so erhaltene in 1-Stellung unsubstituierte Imidazol-4-yl-Verbindung mittels Alkylierung in eine entsprechend in 1-Stellung alkylierte Imidazol-4-yl-Verbindung übergeführt werden.

Eine Ausgangsverbindung der allgemeinen Formel III erhält man durch Reduktion und Cyclisierung einer vorstehend beschrieben o-Nitro-acylamino-Verbindung.

Eine Ausgangsverbindung der allgemeinen Formeln V, VI, VII und VIII erhält man durch Umsetzung einer Verbindung der allgemeinen Formel III mit einer entsprechenden Verbindung der allgemeinen Formel IV.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[(2-Ethyl-4-methyl-6-(1-methyl-5-fluor-benzimidazol-2-yl)-benzimidazol-1-yl)-methyl]-biphenyl-2-carbonsäure,

B = 4'-[(2-n-Propyl-4-methyl-6-(butansultam-1-yl)-benzimidazol-1-yl)-methyl]-biphenyl-2-carbonsäure,

C = 4'-[(2-Cyclopropyl-4-methyl-6-(imidazo[1,2-a]pyridin-2-yl)-benzimidazol-1-yl)-methyl]-biphenyl-2-carbonsäure,

D = 4'-[(2-Cyclopropyl-4-methyl-6-(5,6,7,8-tetrahydro-imidazo[1,2-a]pyridin-2-yl)-benzimidazol-1-yl)-methyl]-biphenyl-2-carbonsäure und

E = 4'-[(2-Ethoxy-4-methyl-6-(1-methyl-benzimidazol-2-yl)-benzimidazol-1-yl)-methyl]-2-(1H-tetrazol-5-yl)-biphenyl,

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37 °C mit 50 pM [$^{125}I$]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis E zeigen in dem beschriebenen Test folgende $IC_{50}$-Werte:

| Substanz | $IC_{50}$ [nM] |
|---|---|
| A | 7,4 |
| B | 0,9 |
| C | 1,7 |
| D | 1,3 |
| E | 3,3 |

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, der Bulimie, sowie von Störungen kognitiver Funktionen.

Die zur Erzielung einer entsprechenden Wirkung am Erwachsenen erforderliche Dosierung beträgt zweckmäßigerweise bei intravenöser Gabe 0,5 bis 100 mg, vorzugsweise 1 bis 70 mg, und bei oraler Gabe 0,1 bis 200 mg, vorzugsweise 1 bis 100 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, ACE-Hemmer, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung,

also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel 1

4'-[(2-Ethyl-4-methyl-6-(1-methyl-5-fluor-benzimidazol-2-yl)-benzimidazol-1-yl)-methyl]-biphenyl-2-carbonsäure

a) 2-Ethyl-4-methyl-6-(1-methyl-5-fluor-benzimidazol-2-yl)-benzimidazol

Eine Mischung aus 4,5 g (21 mMol) 1-Methylamino-2-amino-4-fluor-benzol-dihydrochlorid und 4,3 g (21 mMol) 2-Ethyl-4-methyl-benzimidazol-6-yl-carbonsäure wird vier Stunden lang bei 140 °C in 100 g Polyphosphorsäure gerührt, anschließend in ca. 300 g Eiswasser eingerührt und mit konzentrierter Ammoniaklösung alkalisch gestellt. Das ausgefallene Rohprodukt wird abgesaugt, getrocknet und anschließend durch Säulenchromatographie (300 g Kieselgel; Methylenchlorid/Ethanol = 95:5) gereinigt.
Ausbeute: 3,1 g (48 % der Theorie),
$R_f$-Wert: 0,24 (Kieselgel; Methylenchlorid/Ethanol = 19:1)

b) 4'-[(2-Ethyl-4-methyl-6-(1-methyl-5-fluor-benzimidazol-2-yl)-benzimidazol-1-yl)-methyl]-biphenyl-2-carbonsäure-tert.butylester

Zu einer Lösung von 1,55 g (5 mMol) 2-Ethyl-4-methyl-6-(1-methyl-5-fluor-benzimidazol-2-yl)-benzimidazol in 30 ml Dimethylsulfoxid werden 616 mg (5,5 mMol) Kalium-tert.butylat gegeben und 15 Minuten bei Raumtemperatur gerührt. Dann werden 1,9 g (5,5 mMol) 4'-Brommethyl-biphenyl-2-carbonsäure-tert.butylester hinzugegeben und weitere 20 Stunden bei Raumtemperatur gerührt. Anschließend wird das Gemisch in ca. 80 ml gesättigte Natriumchlorid-Lösung eingerührt, das ausgefallene Rohprodukt abgesaugt und durch Säulenchromatographie (150 g Kieselgel; Laufmittel: Methylenchlorid/Ethanol = 98:2) gereinigt.
Ausbeute: 1,4 g (50 % der Theorie),
$R_f$-Wert: 0,47 (Kieselgel; Methylenchlorid/Ethanol = 19:1)

c) 4'-[(2-Ethyl-4-methyl-6-(1-methyl-5-fluor-benzimidazol-2-yl)-benzimidazol-1-yl)-methyl]-biphenyl-2-carbonsäure

Eine Lösung von 1,4 g (2,4 mMol) 4'-[(2-Ethyl-4-methyl-6-(1-methyl-5-fluor-benzimidazol-2-yl)-benzimidazol-1-yl)-methyl]-biphenyl-2-carbonsäure-tert.butylester und 15 ml Trifluoressigsäure in 30 ml Methylenchlorid wird 14 Stunden lang bei Raumtemperatur gerührt, anschließend eingedampft, der Rückstand mit ca. 30 ml Wasser versetzt und mit 2N Natronlauge alkalisch gestellt. Nach zweimaligem Extrahieren mit je 30 ml Diethylether wird die wäßrige Phase mit 20%iger Zitronensäure angesäuert. Das dabei ausgefallene Rohprodukt wird abgesaugt und durch Säulenchromatographie (100 g Kieselgel; Laufmittel: Methylenchlorid/Ethanol = 96:4) gereinigt.
Ausbeute: 850 mg (69 % der Theorie),
Schmelzpunkt: 246-248 °C

| $C_{32}H_{27}FN_4O_2$ (518,60) | | | | | | |
|---|---|---|---|---|---|---|
| Ber.:<br>Gef.: | C | 74,11<br>73,95 | H | 5,25<br>5,34 | N | 10,80<br>10,80 |

Massenspektrum: m/e = 518

Beispiel 2

4'-[(2-n-Propyl-4-methyl-6-(butansultam-1-yl)-benzimidazol-1-yl)-methyl]-biphenyl-2-carbonsäure

Hergestellt analog Beispiel 1 aus 4'-[(2-n-Propyl-4-methyl-6-(butansultam-1-yl)-benzimidazol-1-yl)-methyl]-biphenyl-2-carbonsäure-tert.butylester und Trifluoressigsäure.

Ausbeute: 87 % der Theorie,
Schmelzpunkt: 269-271 ° C

| $C_{29}H_{31}N_3O_4S$ (517,65) | | | | | | |
|---|---|---|---|---|---|---|
| Ber.:<br>Gef.: | C | 67,29<br>67,56 | H | 6,04<br>6,12 | N | 8,12<br>8,28 |

$R_f$-Wert: 0,39 (Kieselgel; Methylenchlorid/Ethanol = 9:1)
Massenspektrum: m/e = 517

Beispiel 3

4'-[(2-Cyclopropyl-4-methyl-6-(imidazo[1,2-a]pyridin-2-yl)-benzimidazol-1-yl)-methyl]-biphenyl-2-carbonsäure

Hergestellt analog Beispiel 1 aus 4'-[(2-Cyclopropyl-4-methyl-6-(imidazo[1,2-a]pyridin-2-yl)-benzimidazol-1-yl)-methyl]-biphenyl-2-carbonsäure-tert.butylester und Trifluoressigsäure.
Ausbeute: 50 % der Theorie,
Schmelzpunkt: 245-248 ° C

| $C_{32}H_{26}N_4O_2$ (498,59) | | | | | | |
|---|---|---|---|---|---|---|
| Ber.:<br>Gef.: | C | 77,09<br>76,88 | H | 5,26<br>5,37 | N | 11,24<br>11,30 |

$R_f$-Wert: 0,63 (Kieselgel; Methylenchlorid/Ethanol = 9:1)
Massenspektrum: m/e = 498

Beispiel 4

4'-[(2-Cyclopropyl-4-methyl-6-(5,6,7,8-tetrahydro-imidazo[1,2-a]pyridin-2-yl)-benzimidazol-1-yl)-methyl]-biphenyl-2-carbonsäure

Hergestellt analog Beispiel 1 aus 4'-[(2-Cyclopropyl-4-methyl-6-(5,6,7,8-tetrahydro-imidazo[1,2-a]pyridin-2-yl)-benzimidazol-1-yl)-methyl]-biphenyl-2-carbonsäure-tert.butylester und Trifluoressigsäure.
Ausbeute: 53 % der Theorie,
Schmelzpunkt: 310-312 ° C

| $C_{32}H_{30}N_4O_2$ (502,62) | | | | | | |
|---|---|---|---|---|---|---|
| Ber.:<br>Gef.: | C | 76,47<br>76,23 | H | 6,02<br>5,97 | N | 11,15<br>10,85 |

$R_f$-Wert: 0,17 (Kieselgel; Methylenchlorid/Ethanol = 9:1)
Massenspektrum: m/e = 502

Beispiel 5

4'-[(2-Ethyl-4-methyl-6-(1-methyl-5-fluor-benzimidazol-2-yl)-benzimidazol-1-yl)-methyl]-2-(1H-tetrazol-5-yl)-biphenyl

a) 4'-[(2-Ethyl-4-methyl-6-(1-methyl-5-fluor-benzimidazol-2-yl)-benzimidazol-1-yl)-methyl]-2-cyano-biphenyl

Zu einer Lösung von 1,55 g (5 mMol) 2-Ethyl-4-methyl-6-(1-methyl-5-fluor-benzimidazol-2-yl)-benzimidazol in 30 ml Dimethylsulfoxid werden 616 mg (5,5 mMol) Kalium-tert.butylat gegeben und 15 Minuten bei Raumtemperatur gerührt. Dann werden 1,5 g (5,5 mMol) 4'-Brommethyl-2-cyano-biphenyl hinzugegeben und weitere 20 Stunden bei Raumtemperatur gerührt. Anschließend wird das Gemisch in ca. 80 ml

gesättigte Natriumchlorid-Lösung eingerührt, das ausgefallene Rohprodukt abgesaugt und durch Säulenchromatographie (150 g Kieselgel; Laufmittel: Methylenchlorid/Ethanol = 97:3) gereinigt.
Ausbeute: 1,9 g (76 % der Theorie),
$R_f$-Wert: 0,43 (Kieselgel; Methylenchlorid/Ethanol = 19:1)

b) 4'-[(2-Ethyl-4-methyl-6-(1-methyl-5-fluor-benzimidazol-2-yl)-benzimidazol-1-yl)-methyl]-2-(1H-tetrazol-5-yl)-biphenyl

Eine Lösung von 1,9 g (3,8 mMol) 4'-[(2-Ethyl-4-methyl-6-(1-methyl-5-fluor-benzimidazol-2-yl)-benzimidazol-1-yl)-methyl]-2-cyano-biphenyl, 4,1 g (76 mMol) Ammoniumchlorid und 4,9 g (76 mMol) Natriumazid in 30 ml Dimethylformamid wird 15 Stunden lang auf 140 °C erhitzt, dann nochmals 2,0 g Ammoniumchlorid und 2,4 g Natriumazid hinzugefügt und weitere vier Stunden auf 140 °C erhitzt. Anschließend wird die Lösung in ca. 80 ml gesättigte Natriumchlorid-Lösung eingerührt, das ausgefallene Rohprodukt abgesaugt und durch Säulenchromatographie (150 g Kieselgel; Laufmittel: Methylenchlorid/Ethanol = 19:1) gereinigt.
Ausbeute: 1,25 g (61 % der Theorie),
Schmelzpunkt: 267-269 °C

| $C_{32}H_{27}FN_8$ (542,60) | | | | | | |
|---|---|---|---|---|---|---|
| Ber.:<br>Gef.: | C | 70,84<br>70,52 | H | 5,02<br>5,04 | N | 20,65<br>20,82 |

$R_f$-Wert: 0,60 (Kieselgel; Methylenchlorid/Ethanol = 9:1) Massenspektrum: m/e = 542

## Beispiel 6

### 4'-[(2-Ethoxy-4-methyl-6-(1-methyl-benzimidazol-2-yl)-benzimidazol-1-yl)-methyl]-2-(1H-tetrazol-5-yl)-biphenyl

a) 4'-[(2-Ethoxy-4-methyl-6-(1-methyl-benzimidazol-2-yl)-benzimidazol-1-yl)-methyl]-2-(2-triphenylmethyl-tetrazol-5-yl)-biphenyl

Zu einer Lösung von 570 mg (1,86 mMol) 2-Ethoxy-4-methyl-6-(1-methyl-benzimidazol-2-yl)-benzimidazol in 20 ml Dimethylsulfoxid werden 224 mg (2,0 mMol) Kalium-tert.butylat gegeben und 15 Minuten lang bei Raumtemperatur gerührt. Dann werden 1,11 g (2,0 mMol) 4'-Brommethyl-2-(2-triphenylmethyl-tetrazol-5-yl)-biphenyl hinzugegeben und weitere drei Stunden bei Raumtemperatur gerührt. Anschließend wird das Gemisch in ca. 50 ml gesättigte Natriumchlorid-Lösung eingerührt, das ausgefallene Rohprodukt abgesaugt und durch Säulenchromatographie (100 g Kieselgel; Laufmittel: Essigester/Petrolether = 4:1) gereinigt.
Ausbeute: 860 mg (59 % der Theorie),
$R_f$-Wert: 0,56 (Kieselgel; Essigester/Petrolether = 4:1)

b) 4'-[(2-Ethoxy-4-methyl-6-(1-methyl-benzimidazol-2-yl)-benzimidazol-1-yl)-methyl]-2-(1H-tetrazol-5-yl)-biphenyl

Ein Gemisch aus 830 mg (1,06 mMol) 4'-[(2-Ethoxy-4-methyl-6-(1-methyl-benzimidazol-2-yl)-benzimidazol-1-yl)-methyl]-2-(2-triphenylmethyl-tetrazol-5-yl)-biphenyl, 2,5 ml 1N Natronlauge und 20 ml Ethanol wird 2 Stunden lang bei 80 °C gerührt. Die Lösung wird dann eingedampft, der Rückstand mit ca. 30 ml Wasser versetzt und mit Eisessig schwach sauer gestellt. Sodann wird dreimal mit je ca. 20 ml Methylenchlorid extrahiert, die vereinigten organischen Extrakte mit 20 ml Wasser gewaschen und eingeengt. Das so erhaltene Rohprodukt wird durch Säulenchromatographie (50 g Kieselgel; Methylenchlorid/Ethanol = 97:3) gereinigt.
Ausbeute: 430 mg (75 % der Theorie),
Schmelzpunkt: 194-197 °C

| $C_{32}H_{28}N_8O$ (540,60) | | | | | | |
|---|---|---|---|---|---|---|
| Ber.: <br> Gef.: | C | 71,10 <br> 69,99 | H | 5,22 <br> 5,36 | N | 20,73 <br> 20,54 |

Massenspektrum: m/e = 540

Beispiel 7

4'-[(2-Ethoxy-4-methyl-6-(imidazo[1,2-a]pyridin-2-yl)-benzimidazol-1-yl)-methyl]-biphenyl-2-carbonsäure-hydrat

Hergestellt analog Beispiel 1 aus 4'-[(2-Ethoxy-4-methyl-6-(imidazo[1,2-a)pyridin-2-yl)-benzimidazol-1-yl)-methyl]-biphenyl-2-carbonsäure-tert.butylester und Trifluoressigsäure in Methylenchlorid.
Ausbeute: 72 % der Theorie,
Schmelzpunkt: 207-209 ° C

| $C_{31}H_{26}N_4O_3 \times H_2O$ (520,60) | | | | | | |
|---|---|---|---|---|---|---|
| Ber.: <br> Gef.: | C | 71,52 <br> 71,22 | H | 5,42 <br> 5,37 | N | 10,76 <br> 10,76 |

$R_f$-Wert: 0,36 (Kieselgel; Methylenchlorid/Ethanol = 19:1)

Beispiel 8

Gemisch aus
4'-[(2-n-Propyl-4-methyl-6-(butansultam-1-yl)-benzimidazol-1-yl)-methyl]-2-[1-(pivaloyloxymethyl)-tetrazol-5-yl]-biphenyl und
4'-[(2-n-Propyl-4-methyl-6-(butansultam-1-yl)-benzimidazol-1-yl)-methyl]-2-[2-(pivaloyloxymethyl)-tetrazol-5-yl]-biphenyl

Eine Lösung von 400 mg (0,74 mMol) 4'-[(2-n-Propyl-4-methyl-6-(butansultam-1-yl)-benzimidazol-1-yl)-methyl]-2-(1H-tetra zol-5-yl)-biphenyl, 0,16 ml (1,1 mMol) Pivalinsäure-chlormethylester und 194 mg (1,1 mMol) Kaliumcarbonat-dihydrat in 10 ml Dimethylformamid wird 14 Stunden lang bei Raumtemperatur gerührt, dann in ca. 50 ml gesättigte Natriumchloridlösung eingerührt und dreimal mit je ca. 20 ml Methylenchlorid extrahiert. Die vereinigten organischen Extrakte werden mit Wasser gewaschen und eingeengt. Das so erhaltene Rohprodukt wird durch Säulenchromatographie (50 g Kieselgel; Laufmittel: Methylenchlorid/Ethanol = 98:2) gereinigt.
Ausbeute: 400 mg (82 % der Theorie),
Schmelzpunkt: amorph

| $C_{35}H_{41}N_7O_4S$ (655,80) | | | | | | | | |
|---|---|---|---|---|---|---|---|---|
| Ber.: <br> Gef.: | C | 64,10 <br> 63,99 | H | 6,30 <br> 6,22 | N | 14,95 <br> 14,80 | S | 4,88 <br> 5,03 |

$R_f$-Wert: 0,46 (Kieselgel; Methylenchlorid/Ethanol = 19:1)

Beispiel 9

Gemisch aus
4'-[(2-n-Propyl-4-methyl-6-(5,6,7,8-tetrahydro-imidazo[1,2-a]pyridin-2-yl)-benzimidazol-1-yl)-methyl]-2-[1-(pivaloyloxymethyl)-tetrazol-5-yl]-biphenyl und
4'-[(2-n-Propyl-4-methyl-6-(5,6,7,8-tetrahydro-imidazo[1,2-a]pyridin-2-yl)-benzimidazol-1-yl)-methyl]-2-[2-(pivaloyloxymethyl)-tetrazol-5-yl]-biphenyl

Hergestellt analog Beispiel 8 aus 4'-[(2-n-Propyl-4-methyl-6-(5,6,7,8-tetrahydro-imidazo[1,2-a]-pyridin-2-yl)-benzimidazol-1-yl)-methyl]-2-(1H-tetrazol-5-yl)-biphenyl und Pivalinsäure-Chlormethylester.
Ausbeute: 75 % der Theorie,
Schmelzpunkt: 203-205 ° C

| $C_{38}H_{42}N_8O_2$ (642,80) | | | | | | |
|---|---|---|---|---|---|---|
| Ber.:<br>Gef.: | C | 71,00<br>70,85 | H | 6,59<br>6,63 | N | 17,43<br>17,43 |

$R_f$-Wert: 0,43 (Kieselgel; Methylenchlorid/Ethanol = 19:1)
Massenspektrum: m/e = 642

Beispiel 10

4'-[(2-n-Propyl-4-methyl-6-(5,6,7,8-tetrahydro-imidazo[1,2-a]pyridin-2-yl)-benzimidazol-1-yl)-methyl]-2-[1-(cyclohexyloxycarbonyloxy)-ethyloxycarbonyl]-biphenyl

Eine Lösung von 504 mg (1,0 mMol) 4'-[(2-n-Propyl-4-methyl-6-(5,6,7,8-tetrahydro-imidazo[1,2-a]-pyridin-2-yl)-benzimidazol-1-yl)-methyl]-biphenyl-2-carbonsäure, 600 mg 1-(Cyclohexyloxycarbonyloxy)-ethyljodid und 350 mg Kaliumcarbonat in 25 ml Dimethylsulfoxid wird 14 Stunden lang bei Raumtemperatur gerührt, dann in ca. 70 ml gesättigte Natriumchloridlösung eingerührt und dreimal mit je ca. 30 ml Essigester extrahiert. Die vereinigten organischen Extrakte werden mit Wasser gewaschen und eingeengt. Das so erhaltene Rohprodukt wird durch Säulenchromatographie (100 g Kieselgel; Laufmittel: Methylenchlorid/Ethanol = 98:2) gereinigt.
Ausbeute: 325 mg (48 % der Theorie),
Schmelzpunkt: 162-164 ° C

| $C_{41}H_{46}N_4O_5$ (674,85) | | | | | | |
|---|---|---|---|---|---|---|
| Ber.:<br>Gef.: | C | 72,97<br>72,63 | H | 6,87<br>6,77 | N | 8,30<br>8,17 |

$R_f$-Wert: 0,52 (Kieselgel; Methylenchlorid/Ethanol = 19:1)
Massenspektrum: m/e = 674

Beispiel 11

4'-[(2-n-Propyl-4-methyl-6-(5,6,7,8-tetrahydro-imidazo[1,2-a]pyridin-2-yl)-benzimidazol-1-yl)-methyl]-2-(pivaloyloxymethyloxycarbonyl)-biphenyl

Hergestellt analog Beispiel 8 aus 4'-[(2-n-Propyl-4-methyl-6-(5,6,7,8-tetrahydro-imidazo[1,2-a]-pyridin-2-yl)-benzimidazol-1-yl)-methyl]-biphenyl-2-carbonsäure und Pivalinsäurechlormethylester in Dimethylformamid.
Ausbeute: 76 % der Theorie,
Schmelzpunkt: 142-144 ° C

| $C_{38}H_{42}N_4O_4$ (618,79) | | | | | | |
|---|---|---|---|---|---|---|
| Ber.:<br>Gef.: | C | 73,76<br>73,60 | H | 6,84<br>6,92 | N | 9,09<br>9,17 |

$R_f$-Wert: 0,40 (Kieselgel; Methylenchlorid/Ethanol = 19:1)
Massenspektrum: m/e = 618

Beispiel 12

4'-[(2-n-Propyl-4-methyl-6-(butansultam-1-yl)-benzimidazol-1-yl)-methyl]-2-(pivaloyloxymethyloxycarbonyl)-biphenyl

Hergestellt analog Beispiel 8 aus 4'-[(2-n-Propyl-4-methyl-6-(butansultam-1-yl)-benzimidazol-1-yl)-methyl]-biphenyl-2-carbonsäure und Pivalinsäure-chlormethylester in Dimethylformamid.
Ausbeute: 70 % der Theorie,
Schmelzpunkt: Öl

| $C_{35}H_{41}N_3O_6S$ (631,80) | | | | | | | | |
|---|---|---|---|---|---|---|---|---|
| Ber.:<br>Gef.: | C | 66,54<br>66,21 | H | 6,54<br>6,67 | N | 6,65<br>6,54 | S | 5,08<br>5,34 |

$R_f$-Wert: 0,49 (Kieselgel; Methylenchlorid/Ethanol = 19:1)
Massenspektrum: m/e = 631

Beispiel 13

4'-[(2-n-Propyl-4-methyl-6-(5,6,7,8-tetrahydro-imidazo[1,2-a]pyridin-2-yl)-benzimidazol-1-yl)-methyl]-2-[1-(ethoxycarbonyloxymethyloxy)-carbonyl]-biphenyl

Hergestellt analog Beispiel 8 aus 4'-[(2-n-Propyl-4-methyl-6-(5,6,7,8-tetrahydro-imidazo[1,2-a]-pyridin-2-yl)-benzimidazol-1-yl)-methyl]-biphenyl-2-carbonsäure und 1-(Ethoxycarbonyloxy)-methylchlorid in Dimethylformamid.
Ausbeute: 38,5 % der Theorie,
Schmelzpunkt: 123-125 ° C

| $C_{37}H_{40}N_4O_5$ (620,76) | | | | | | |
|---|---|---|---|---|---|---|
| Ber.:<br>Gef.: | C | 71,59<br>71,57 | H | 6,50<br>6,58 | N | 9,03<br>9,03 |

$R_f$-Wert: 0,33 (Kieselgel; Methylenchlorid/Ethanol = 19:1)
Massenspektrum: m/e = 620

Beispiel 14

4'-[(2-Ethoxy-4-methyl-6-(imidazo[1,2-a]pyridin-2-yl)-benzimidazol-1-yl)-methyl]-2-(1H-tetrazol-5-yl)-biphenyl

Hergestellt analog Beispiel 6 aus 4'-[(2-Ethoxy-4-methyl-6-(imidazo[1,2-a]-pyridin-2-yl)-benzimidazol-1-yl)-methyl]-2-(2-triphenylmethyl-tetrazol-5-yl)-biphenyl und Natronlauge in Ethanol.

Beispiel 15

4'-[(2-Ethoxy-4-methyl-6-(5,6,7,8-tetrahydro-imidazo[1,2-a]pyridin-2-yl)-benzimidazol-1-yl)-methyl]-2-(1H-tetrazol-5-yl)-biphenyl

Hergestellt analog Beispiel 6 aus 4'-[(2-Ethoxy-4-methyl-6-(5,6,7,8-tetrahydro-imidazo[1,2-a]-pyridin-2-yl)-benzimidazol-1-yl)-methyl]-2-(2-triphenylmethyl-tetrazol-5-yl)-biphenyl und Natronlauge in Ethanol.

Beispiel 16

4'-[(2-Ethoxy-4-methyl-6-(5,6,7,8-tetrahydro-imidazo[1,2-a]pyridin-2-yl)-benzimidazol-1-yl)-methyl]-biphenyl-2-carbonsäure

Hergestellt analog Beispiel 1 aus 4'-[(2-Ethoxy-4-methyl-6-(5,6,7,8-tetrahydro-imidazo[1,2-a]-pyridin-2-yl)-benzimidazol-1-yl)-methyl]-biphenyl-2-carbonsäure-tert.butylester und Trifluoressigsäure in Methylenchlorid.
Ausbeute: 63 % der Theorie,
Schmelzpunkt: 238-240 ° C
$R_f$-Wert: 0,62 (Kieselgel; Methylenchlorid/Ethanol = 9:1)

Beispiel 17

4'-[(2-Ethyl-4-methyl-6-(butansultam-1-yl)-benzimidazol-1-yl)-methyl]-biphenyl-2-carbonsäure

Hergestellt analog Beispiel 1 aus 4'-[(2-Ethyl-4-methyl-6-(butansultam-1-yl)-benzimidazol-1-yl)-methyl]-biphenyl-2-carbonsäure-tert.butylester und Trifluoressigsäure in Methylenchlorid.
Ausbeute: 68 % der Theorie,
Schmelzpunkt: > 240 ° C

| $C_{28}H_{29}N_3O_4S$ (503,60) | | | | | | |
|---|---|---|---|---|---|---|
| Ber.:<br>Gef.: | C | 66,77<br>66,57 | H | 5,80<br>5,69 | N | 8,34<br>8,30 |

$R_f$-Wert: 0,36 (Kieselgel; Methylenchlorid/Ethanol = 9:1)

Beispiel 18

4'-[(2-Ethyl-4-methyl-6-(3-chlor-5,6,7,8-tetrahydro-imidazo[1,2-a]pyridin-2-yl)-benzimidazol-1-yl)-methyl]-biphenyl-2-carbonsäure

Hergestellt analog Beispiel 1 aus 4'-[(2-Ethyl-4-methyl-6-(3-chlor-5,6,7,8-tetrahydro-imidazo[1,2-a]-pyridin-2-yl)-benzimidazol-1-yl)-methyl]-biphenyl-2-carbonsäure-tert.butylester und Trifluoressigsäure in Methylenchlorid.
Ausbeute: 43 % der Theorie,
Schmelzpunkt: 295-297 ° C

| $C_{31}H_{29}ClN_4O_2$ (525,06) | | | | | | | | |
|---|---|---|---|---|---|---|---|---|
| Ber.:<br>Gef.: | C | 70,91<br>70,81 | H | 5,57<br>5,54 | N | 10,67<br>10,55 | Cl | 6,75<br>6,83 |

Bei den nachfolgenden pharmazeutischen Anwendungsbeispielen kann als Wirksubstanz jede geeignete Verbindung der Formel I, insbesondere diejenigen, in denen $R_4$ eine in 1- oder 2-Stellung durch eine $R_a$-CO-O-$CH_2$-Gruppe substituierte Tetrazolylgruppe, eine Carboxy-, 1H-Tetrazolyl-, $R_a$O-CO-, $R_b$-CO-O-($R_c$CH)-O-CO- oder $R_b$O-CO-O-($R_c$CH)-O-CO-Gruppe darstellt, eingesetzt werden:

Beispiel I

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1.5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel IV

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1.0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1.2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45°C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 °C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40 °C wird die gemahlene Wirksubstanz in der Schmelze homogen dispergiert. Es wird auf 38 °C abgekühlt und in schwach vorgekühlte Suppositorienformen ausgegossen.

**Patentansprüche**

**1.** Benzimidazole der allgemeinen Formel

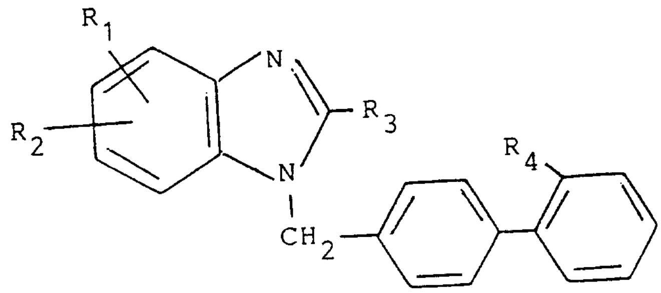

, (I)

in der

$R_1$ ein Wasserstoff-, Fluor-, Chlor- oder Bromatom, eine Alkylgruppe mit 1 bis 3 Kohlenstoffatomen, eine Cycloalkylgruppe mit 3 bis 7 Kohlenstoffatomen, eine Fluormethyl-, Difluormethyl- oder Trifluormethylgruppe,

$R_2$ eine Phthalimino- oder Homophthaliminogruppe, wobei eine Carbonylgruppe in einer Phthaliminogruppe durch eine Methylengruppe ersetzt sowie eine Methylengruppe in einer Homophthaliminogruppe durch eine oder zwei Alkylgruppen mit jeweils 1 bis 3 Kohlenstoffatomen substituiert sein kann,

eine gegebenenfalls durch eine oder zwei Alkylgruppen mit jeweils 1 bis 3 Kohlenstoffatomen substituierte 5-, 6- oder 7-gliedrige Alkyleniminogruppe, in welcher eine Methylengruppe durch eine Carbonyl- oder Sulfonylgruppe ersetzt ist,

eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,

eine gegebenenfalls in 1-Stellung durch eine Alkylgruppe mit 1 bis 6 Kohlenstoffatomen oder eine Cycloalkylgruppe mit 3 bis 5 Kohlenstoffatomen substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann,

eine Imidazo[2,1-b]thiazol-6-yl-, Imidazo[1,2-a]pyridin-2-yl-, 5,6,7,8-Tetrahydro-imidazo[1,2-a]pyridin-2-yl-, 3-Chlor-5,6,7,8-tetrahydro-imidazo[1,2-a]pyridin-2-yl-, Imidazo[1,2-a]pyrimidin-2-yl-, Imidazo[4,5-b]-pyridin-2-yl-, Imidazo[4,5-c]pyridin-2-yl-, Imidazo[1,2-c]pyrimidin-2-yl-, Imidazo[1,2-a]pyrazin-2-yl-, Imidazo[1,2-b]pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl- oder Imidazo[4,5-d]pyridazin-2-yl-gruppe, einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonylgruppe ersetzt sein kann, oder

eine $R_7$-$NR_6$-CO-$NR_5$-Gruppe, in der

$R_5$ ein Wasserstoffatom, eine Alkylgruppe mit 1 bis 5 Kohlenstoffatomen, eine Cyclohexyl- oder Benzylgruppe,

$R_6$ ein Wasserstoffatom, eine Alkylgruppe mit 1 bis 6 Kohlenstoffatomen, eine Allyl-, Cyclohexyl, Benzyl- oder Phenylgruppe,

$R_7$ ein Wasserstoffatom oder eine Alkylgruppe mit 1 bis 3 Kohlenstoffatomen oder

$R_6$ und $R_7$ zusammen mit dem dazwischen liegenden Stickstoffatom eine unverzweigte cyclische Alkyleniminogruppe mit 4 bis 6 Kohlenstoffatomen oder eine Morpholinogruppe oder

$R_5$ und $R_6$ zusammen eine Alkylengruppe mit 2 oder 3 Kohlenstoffatomen darstellen,

$R_3$ eine Alkylgruppe mit 1 bis 5 Kohlenstoffatomen, eine Cycloalkylgruppe mit 3 bis 5 Kohlenstoffatomen oder eine Alkoxygruppe mit 1 bis 3 Kohlenstoffatomen und

$R_4$ eine in 1- oder 2-Stellung durch eine $R_a$-CO-O-$CH_2$-Gruppe substituierte Tetrazolylgruppe, eine $R_b$-CO-O-($R_c$CH)-O-CO-, $R_a$O-CO- oder $R_b$O-CO-O-($R_c$CH)-O-CO-Gruppe, wobei

$R_a$ eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,

$R_b$ eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe und

$R_c$ ein Wasserstoffatom oder eine Methylgruppe darstellen,

oder auch, wenn

(i) $R_1$ und $R_2$ wie vorstehend erwähnt definiert sind und $R_3$ eine Alkoxygruppe darstellt, eine Carboxy-, 1H-Tetrazolyl- oder 2H-Tetrazolylgruppe oder, wenn

(ii) $R_1$ wie vorstehend erwähnt definiert ist, $R_2$ mit Ausnahme der 1-Methyl-benzimidazol-2-yl-gruppe wie vorstehend erwähnt definiert ist und $R_3$ eine Cyclopropylgruppe darstellt, eine Carboxygruppe oder, wenn

(iii) $R_1$ und $R_3$ wie vorstehend erwähnt definiert sind und $R_2$ eine Butansultam-1-yl-gruppe darstellt, eine Carboxygruppe oder, wenn

(iv) $R_1$ wie vorstehend erwähnt definiert ist, $R_2$ eine 1-Methyl-5-fluor-benzimidazol-2-yl-gruppe und $R_3$ eine Ethylgruppe darstellen, eine Carboxy-, 1H-Tetrazolyl- oder 2H-Tetrazolylgruppe bedeuten,

deren Tautomere und deren Salze.

2. Benzimidazole der allgemeinen Formel I gemäß Anspruch 1, in der
$R_1$ ein Wasserstoffatom oder in 4-Position ein Chloratom, eine Alkylgruppe mit 1 bis 3 Kohlenstoffatomen oder eine Trifluormethylgruppe,
$R_2$ eine Phthalimino- oder Homophthaliminogruppe, wobei eine Carbonylgruppe in einer Phthaliminogruppe durch eine Methylengruppe ersetzt sein kann,
eine 5-, 6- oder 7-gliedrige Alkyleniminogruppe, in welcher eine Methylengruppe durch eine Carbonyl- oder Sulfonylgruppe ersetzt ist,
eine gegebenenfalls durch eine Methyl- oder Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, 3-Chlor-5,6,7,8-tetrahydro-imidazo[1,2-a]pyridin-2-yl-, Imidazo[1,2-a]pyrimidin-2-yl-, Imidazo[4,5-b]pyridin-2-yl-, Imidazo[4,5-c]-pyridin-2-yl-, Imidazo[1,2-c]pyrimidin-2-yl-, Imidazo[1,2-a]pyrazin-2-yl-, Imidazo[1,2-b]pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl- oder Imidazo[4,5-d]pyridazin-2-yl-gruppe oder
eine $R_7$-$NR_6$-CO-$NR_5$-Gruppe, in der
$R_5$ ein Wasserstoffatom, eine Alkylgruppe mit 1 bis 5 Kohlenstoffatomen, eine Cyclohexyl- oder Benzylgruppe,
$R_6$ ein Wasserstoffatom, eine Alkylgruppe mit 1 bis 6 Kohlenstoffatomen, eine Allyl-, Cyclohexyl-, Benzyl- oder Phenylgruppe,
$R_7$ ein Wasserstoffatom oder eine Alkylgruppe mit 1 bis 3 Kohlenstoffatomen oder
$R_6$ und $R_7$ zusammen mit dem dazwischen liegenden Stickstoffatom eine geradkettige Alkyleniminogruppe mit 4 bis 6 Kohlenstoffatomen oder eine Morpholinogruppe oder
$R_5$ und $R_6$ zusammen eine Alkylengruppe mit 2 oder 3 Kohlenstoffatomen darstellen,
$R_3$ eine Alkylgruppe mit 1 bis 5 Kohlenstoffatomen, eine Cycloalkylgruppe mit 3 bis 5 Kohlenstoffatomen oder eine Alkoxygruppe mit 2 bis 3 Kohlenstoffatomen und
$R_4$ eine in 1- oder 2-Stellung durch eine $R_a$-CO-O-$CH_2$-Gruppe substituierte Tetrazolylgruppe, eine $R_b$-CO-O-($R_c$CH)-O-CO-, $R_a$O-CO- oder $R_b$O-CO-O-($R_c$CH)-O-CO-Gruppe, wobei
$R_a$ eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
$R_b$ eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe und
$R_c$ ein Wasserstoffatom oder eine Methylgruppe darstellen,
oder auch, wenn
(i) $R_1$ und $R_2$ wie vorstehend erwähnt definiert sind und $R_3$ eine Alkoxygruppe darstellt, eine Carboxy-, 1H-Tetrazolyl- oder 2H-Tetrazolylgruppe oder, wenn
(ii) $R_1$ wie vorstehend erwähnt definiert ist, $R_2$ mit Ausnahme der 1-Methyl-benzimidazol-2-yl-gruppe wie vorstehend erwähnt definiert ist und $R_3$ eine Cyclopropylgruppe darstellt, eine Carboxygruppe oder, wenn
(iii) $R_1$ und $R_3$ wie vorstehend erwähnt definiert sind und $R_2$ eine Butansultam-1-yl-gruppe darstellt, eine Carboxygruppe oder, wenn
(iv) $R_1$ wie vorstehend erwähnt definiert ist, $R_2$ eine 1-Methyl-5-fluor-benzimidazol-2-yl-gruppe und $R_3$ eine Ethylgruppe darstellen, eine Carboxy-, 1H-Tetrazolyl- oder 2H-Tetrazolylgruppe bedeuten,
deren Tautomere und deren Salze.

3. Benzimidazole der allgemeinen Formel I gemäß Anspruch 1, in der
$R_1$ ein Wasserstoffatom oder in 4-Position eine Methylgruppe,
$R_2$ eine Imidazo[1,2-a]pyridin-2-yl-, 5,6,7,8-Tetrahydro-imidazo[1,2-a]pyridin-2-yl-, 3-Chlor-5,6,7,8-tetrahydro-imidazo[1,2-a]pyridin-2-yl-, 1-Methyl-benzimidazol-2-yl-, 1-Methyl-5-fluor-benzimidazol-2-yl- oder Butansultam-1-yl-gruppe,
$R_3$ eine Alkylgruppe mit 2 bis 4 Kohlenstoffatomen, eine Cyclopropylgruppe oder eine Alkoxygruppe mit 2 oder 3 Kohlenstoffatomen und
$R_4$ eine in 1- oder 2-Stellung durch eine $R_a$-CO-O-$CH_2$-Gruppe substituierte Tetrazolylgruppe, eine $R_b$-

CO-O-($R_cCH$)-O-CO-, $R_aO$-CO- oder $R_bO$-CO-O-($R_cCH$)-O-CO-Gruppe bedeuten, wobei

$R_a$ eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl, 3-Phenylpropyl-, Methoxymethyl- oder Cinnamylgruppe,

$R_b$ eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl, 1-Phenylethyl-, 2-Phenylethyl- oder 3-Phenylpropylgruppe und

$R_c$ ein Wasserstoffatom oder eine Methylgruppe bedeuten,

sowie die Verbindungen

4'-[(2-Cyclopropyl-4-methyl-6-(4,5,6,7-tetrahydro-imidazo[1,2-a]pyridin-2-yl)-benzimidazol-1-yl)-methyl]-biphenyl-2-carbonsäure,

4'-[(2-Ethoxy-4-methyl-6-(4,5,6,7-tetrahydro-imidazo[1,2-a]pyridin-2-yl)-benzimidazol-1-yl)-methyl]-biphenyl-2-carbonsäure,

4'-[(2-Ethoxy-4-methyl-6-(4,5,6,7-tetrahydro-imidazo[1,2-a]pyridin-2-yl)-benzimidazol-1-yl)-methyl]-2-(1H-tetrazol-5-yl)-biphenyl,

4'-[(2-Cyclopropyl-4-methyl-6-(imidazo[1,2-a]pyridin-2-yl)-benzimidazol-1-yl)-methyl]-biphenyl-2-carbonsäure,

4'-[(2-Ethoxy-4-methyl-6-(imidazo[1,2-a]pyridin-2-yl)-benzimidazol-1-yl)-methyl]-biphenyl-2-carbonsäure,

4'-[(2-Ethoxy-4-methyl-6-(imidazo[1,2-a]pyridin-2-yl)-benzimidazol-1-yl)-methyl]-2-(1H-tetrazol-5-yl)-biphenyl,

4'-[(2-Ethoxy-4-methyl-6-(1-methyl-benzimidazol-2-yl)-benzimidazol-1-yl)-methyl]-2-(1H-tetrazol-5-yl)-biphenyl,

4'-[(2-Ethyl-4-methyl-6-(1-methyl-5-fluor-benzimidazol-2-yl)-benzimidazol-1-yl)-methyl]-2-(1H-tetrazol-5-yl)-biphenyl,

4'-[(2-Ethyl-4-methyl-6-(1-methyl-5-fluor-benzimidazol-2-yl)-benzimidazol-1-yl)-methyl]-biphenyl-2-carbonsäure und

4'-[(2-n-Propyl-4-methyl-6-(butansultam-1-yl)-benzimidazol-1-yl)-methyl]-biphenyl-2-carbonsäure,

deren Tautomere und deren Salze.

**4.** Folgende Verbindungen der allgemeinen Formel I gemäß Anspruch 1:

(a) 4'-[(2-Ethyl-4-methyl-6-(1-methyl-5-fluor-benzimidazol-2-yl)-benzimidazol-1-yl)-methyl]-biphenyl-2-carbonsäure,

(b) 4'-[(2-n-Propyl-4-methyl-6-(butansultam-1-yl)-benzimidazol-1-yl)-methyl]-biphenyl-2-carbonsäure,

(c) 4'-[(2-Cyclopropyl-4-methyl-6-(imidazo[1,2-a]pyridin-2-yl)-benzimidazol-1-yl)-methyl]-biphenyl-2-carbonsäure,

(d) 4'-[(2-Cyclopropyl-4-methyl-6-(5,6,7,8-tetrahydro-imidazo[1,2-a]pyridin-2-yl)-benzimidazol-1-yl)-methyl]-biphenyl-2-carbonsäure und

(e) 4'-[(2-Ethoxy-4-methyl-6-(1-methyl-benzimidazol-2-yl)-benzimidazol-1-yl)-methyl]-2-(1H-tetrazol-5-yl)-biphenyl,

deren Tautomere und deren Salze.

**5.** Physiologisch verträgliche Salze der Verbindungen nach mindestens einem der Ansprüche 1 bis 4 mit anorganischen oder organischen Säuren oder Basen.

**6.** Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

**7.** Verwendung einer Verbindung nach mindestens einem der Ansprüche 1 bis 5 zur Herstellung eines Arzneimittels mit Angiotensin-antagonistischer Wirkung.

**8.** Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

**9.** Verfahren zur Herstellung der Benzimidazole gemäß den Ansprüchen 1 bis 5, dadurch gekennzeichnet, daß

a) eine gegebenenfalls im Reaktionsgemisch gebildete Verbindung der allgemeinen Formel

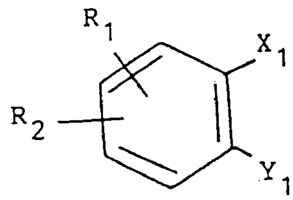 , (II)

in der
$R_1$ und $R_2$ wie in den Ansprüchen 1 bis 4 definiert sind, einer der Reste $X_1$ oder $Y_1$ eine Gruppe der allgemeinen Formel

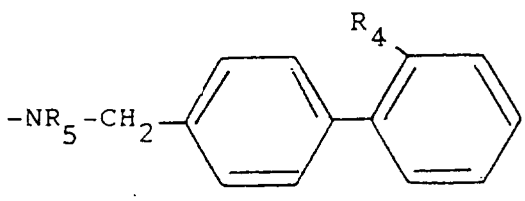

und der andere der Reste $X_1$ oder $Y_1$ eine Gruppe der allgemeinen Formel

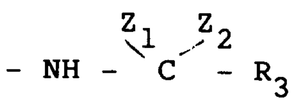

darstellen, wobei
$R_3$ und $R_4$ wie in den Ansprüchen 1 bis 4 definiert sind,
$R_5$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls durch Alkylgruppen mit 1 bis 6 Kohlenstoffatomen substituierte Amino-, Hydroxy- oder Mercaptogruppen oder $Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert und ein gegebenenfalls so erhaltenes N-Oxid reduziert wird oder
b) ein Benzimidazol der allgemeinen Formel

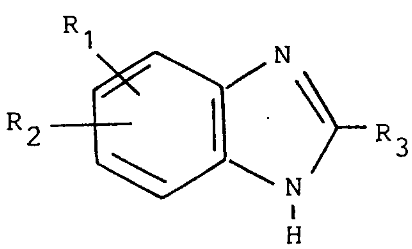 , (III)

in der
$R_1$ bis $R_3$ wie in den Ansprüchen 1 bis 4 definiert sind, mit einer Biphenylverbindung der allgemeinen Formel

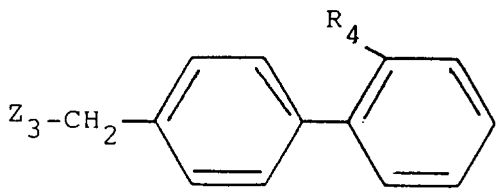

, (IV)

in der

$R_4$ wie in den Ansprüchen 1 bis 4 definiert ist und

$Z_3$ eine nukleophile Austrittsgruppe darstellt, umgesetzt wird oder

c) zur Herstellung einer Verbindung der allgemeinen Formel I, in der $R_4$ eine Carboxygruppe darstellt, eine Verbindung der allgemeinen Formel

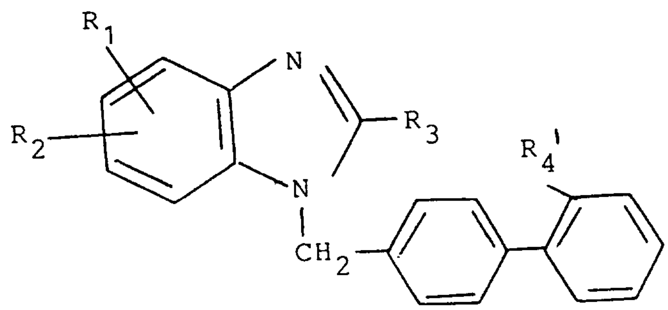

, (V)

in der

$R_1$ bis $R_3$ wie in den Ansprüchen 1 bis 4 definiert sind und

$R_4$' eine mittels Hydrolyse, Thermolyse oder Hydrogenolyse in eine Carboxygruppe überführbare Gruppe darstellt, in eine entsprechende Carboxyverbindung übergeführt wird oder

d) zur Herstellung einer Verbindung der allgemeinen Formel I, in der $R_4$ eine 1H-Tetrazolylgruppe darstellt, ein Schutzrest von einer Verbindung der allgemeinen Formel

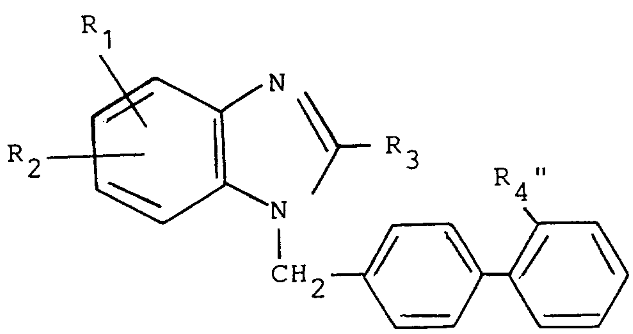

, (VI)

in der

$R_1$, $R_2$ und $R_3$ wie in den Ansprüchen 1 bis 4 definiert sind und

$R_4$" eine in 1- oder 3-Stellung durch einen Schutzrest geschützte 1H-Tetrazolylgruppe darstellt, abgespalten wird oder

e) zur Herstellung einer Verbindung der allgemeinen Formel I, in der $R_4$ eine 1H-Tetrazolylgruppe darstellt, eine Verbindung der allgemeinen Formel

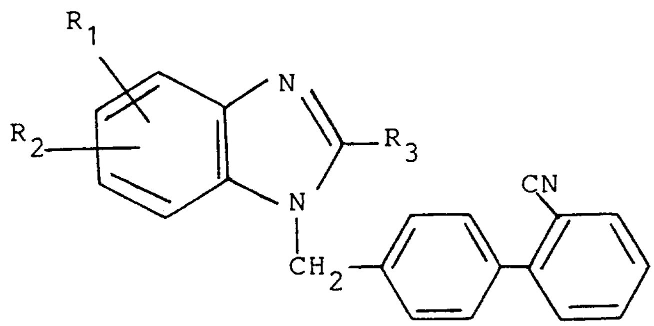

, (VII)

in der

$R_1$ bis $R_3$ wie in den Ansprüchen 1 bis 4 definiert sind, mit Stickstoffwasserstoffsäure oder deren Salzen umgesetzt wird oder

f) zur Herstellung einer Verbindung der allgemeinen Formel I, in der $R_4$ eine in 1- oder 2-Stellung durch eine $R_a$-CO-O-$CH_2$-Gruppe substituierte Tetrazolylgruppe, eine $R_a$O-CO-, $R_b$-CO-O-($R_c$CH)-O-CO- oder $R_b$O-CO-O-($R_c$CH)-O-CO-Gruppe darstellt, eine Verbindung der allgemeinen Formel

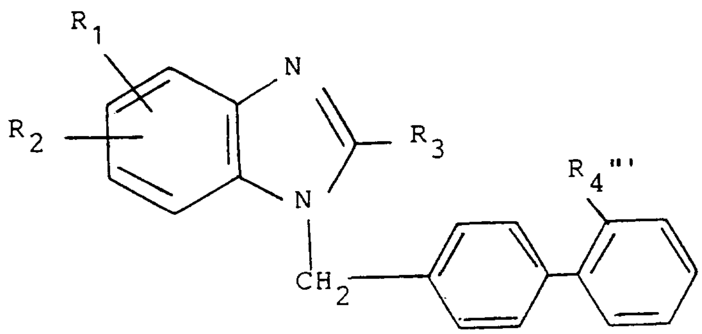

, (VIII)

in der

$R_1$ bis $R_3$ wie in den Ansprüchen 1 bis 4 definiert sind und

$R_4'''$ eine Carboxy-, 1H-Tetrazolyl- oder 2H-Tetrazolylgruppe bedeutet, mit einer Verbindung der allgemeinen Formel

$Z_4$ - $Y_2$ ,(IX)

in der

$Y_2$ eine $R_a$-CO-O-$CH_2$-, $R_b$-CO-O-($R_c$CH)-, $R_b$O-CO-O-($R_c$CH)- oder $R_a$-Gruppe, wobei $R_a$ bis $R_c$ wie in den Ansprüchen 1 bis 4 definiert sind und

$Z_4$ eine nukleophile Austrittsgruppe oder auch, wenn Y eine $R_a$-Gruppe darstellt, eine Hydroxygruppe bedeuten, umgesetzt wird und

erforderlichenfalls ein während der Umsetzungen a) bis f) zum Schutze von reaktiven Gruppen verwendeter Schutzrest abgespalten wird und/oder

gewünschtenfalls anschließend ein so erhaltenes 1-, 3-Isomerengemisch einer Verbindung der allgemeinen Formel I mittels Isomerentrennung in ihr 1- und 3-Isomer aufgetrennt wird oder

eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 93 11 1581

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| X | EP-A-0 468 470 (DR. KARL THOMAE)<br>* Ansprüche 1,7 *<br>--- | 1,7 | C07D235/08<br>C07D235/26<br>C07D471/04<br>C07D417/04<br>C07D403/14<br>C07D417/14<br>A61K31/415<br>A61K31/54<br>A61K31/435<br>//(C07D471/04,<br>235:00,221:00) |
| X | EP-A-0 392 317 (DR. KARL THOMAE)<br>* Ansprüche 1,9 *<br>--- | 1,7 | |
| P,X | EP-A-0 502 314 (DR. KARL THOMAE)<br>* Ansprüche 1,8 *<br>----- | 1,7 | |

RECHERCHIERTE SACHGEBIETE (Int. Cl. 5)

C07D
A61K

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| DEN HAAG | 06 OKTOBER 1993 | VOYIAZOGLOU D. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EP 93 11 1581 -C-

UNVOLLSTÄNDIGE RECHERCHE

Vollständig recherchierte Patentansprüche : 4
Unvollständig recherchierte Patentansprüche : 1-3,5-9

Die Abfassung der Ansprüche ist nicht klar und knapp zu fassen (Art. 83-84, EPA) und enthält eine so grosse Zahl Verbindungen dass eine vollständige Recherche aus ökonomischen Gründen nicht möglich ist (Siehe Richtlinien für die Prüfung im Europaïschen Patentamt, Teil B, Kapittel III,2 (Umfang der Recherche)).
Geleitet durch den Sinn der Anfrage und die Erfinderische Idee als offenbart in die Beschreibung der vorliegende Anfrage, ist die Recherche gegründet auf die Beispiele.